Europäisches Patentamt

European Patent Office

Office européen des brevets

(19)

(11) Veröffentlichungsnummer : **0 235 692 B1**

# (12) EUROPÄISCHE PATENTSCHRIFT

(45) Veröffentlichungstag der Patentschrift :
06.11.91 Patentblatt 91/45

(51) Int. Cl.⁵ : **C07K 5/06, C07K 5/08,**
**A61K 37/02**

(21) Anmeldenummer : **87102369.3**

(22) Anmeldetag : **19.02.87**

(54) **Oligopeptidylnitrilderivate, diese enthaltende Mittel, Verfahren zu ihrer Herstellung und ihre Verwendung.**

(30) Priorität : **28.02.86 DE 3606480**

(43) Veröffentlichungstag der Anmeldung :
**09.09.87 Patentblatt 87/37**

(45) Bekanntmachung des Hinweises auf die
Patenterteilung :
**06.11.91 Patentblatt 91/45**

(84) Benannte Vertragsstaaten :
**AT BE CH DE FR GB IT LI LU NL SE**

(56) Entgegenhaltungen :
**Keine Entgegenhaltungen.**

(73) Patentinhaber : **BEHRINGWERKE**
**Aktiengesellschaft**
**Postfach 1140**
**W-3550 Marburg 1 (DE)**

(72) Erfinder : **Stüber, Werner, Dr.**
**Cölber Weg 12**
**W-3551 Lahntal (DE)**

(74) Vertreter : **Meyer-Dulheuer, Karl-Hermann, Dr.**
**et al**
**HOECHST Aktiengesellschaft Postfach 3540**
**W-6200 Wiesbaden (DE)**

## Beschreibung

Oligopeptidylnitrilderivate, diese enthaltende Mittel, Verfahren zu ihrer Herstellung und ihre Verwendung

Die Erfindung betrifft Oligopeptidylnitrilderivate, die Synthese und Verwendung dieser Verbindungen sowie pharmazeutische Mittel, die diese als Serinproteasen-Inhibitoren wirksamen Verbindungen enthalten.

Die Bezeichnung Oligopeptidylnitrilderivate wird für Derivate einer alpha-Aminosäure gebraucht, deren Carboxygruppe durch eine Cyanidgruppe ersetzt ist, und deren Aminogruppe mit einer alpha-Aminoacyl oder einer peptidylgruppe substituiert ist.

Bekanntlich führen eine ganze Reihe von pathophysiologischen Bedingungen zu einem Verbrauch von AT III, dem wichtigsten Thrombininhibitor im Humanplasma, unter Bildung von Thrombin-AT III-Komplexen. Es kommt zu einem erhöhten Thromboserisiko, wenn der AT III-Spiegel auf unter 75 % der Norm absinkt. Die Therapie von erworbenem und angeborenem AT III-Mangel erfolgt durch Zuführung von AT III, das aus plasma von Blutspendern gewonnen wird. Aufgrund der begrenzten Verfügbarkeit von Humanplasma sind dieser Therapie Grenzen gesetzt. Deshalb ist es wünschenswert, den natürlich vorkommenden Thrombininhibitor durch synthetische Verbindungen zu ersetzen, die in der Lage sind, den Vorgang der Blutgerinnung zu unterbinden oder zu verlangsamen.

Nach dem Stand des Wissens sind solche synthetische Inhibitoren Substrate, die in der Lage sind, die Spezifitätstasche einer protease zu verschließen und damit deren Aktivität zu senken. Zu diesem Zweck eignen sich Peptidderivate des Arginins, da sich diese gut in die Spezifitätstasche von Serinproteasen einfügen lassen. Als besonders günstig hat sich die peptidsequenz D-Phe-Pro-Arg erwiesen, die besonders Thrombin inhibiert.

Eine besondere Bedeutung kommt dabei einer Derivatisierung des C-Terminus am Arginin zu, da hierdurch die Wirksamkeit dieser proteaseinhibitoren stark beeinflußt wird (Annals of the New York Academy of Science (1981), 370, 765-784). Es ist bekannt, daß derartige Derivate als potente Serinproteaseinhibitoren wirken, wenn der C-Terminus des Peptids eine reaktive Gruppe darstellt. Als sehr wirksam haben sich die Formylgruppe und die Chlormethylketongruppe am Arginin erwiesen.

Weitere bekannte Verbindungen, die als Thrombininhibitoren wirksam sind, besitzen eine strukturelle Verwandschaft zum Arginin, beispielsweise Agmatinderivate oder N-alpha-Arylsulfonyl-p-guanidinophenylalaninamide.

Überraschenderweise wurde nun gefunden, daß Verbindungen der Formel I

$$A-R-NH-CHR^1- (CH_2)_a-NHC( = NH)NH_2 \quad I$$

worin

R$^1$ die Cyanidfunktior.

R pro oder D-Phe-Pro,

A ein Wasserstoffatom oder eine in der Peptidchemie übliche Schutzgruppe und

a eine ganze Zahl von 2 bis 5, vorzugsweise 3 oder 4 ist,

in der Lage sind, Serinproteasen zu inhibieren.

Gegenstand der Erfindung ist deshalb eine Verbindung der Formel I mit den angegebenen Definitionen sowie ihre physiologisch verträglichen Salze.

Die erfindungsgemäßen Verbindungen werden nach in der Peptidchemie üblichen Methoden fragment- oder stufenweise mittels geeignet geschützten Aminosäurederivaten unter Zuhilfenahme temporärer Schutzgruppen synthetisiert. Gegebenenfalls werden die Schutzgruppen abgespalten und Salze hergestellt. Die Cyanidfunktion wird durch Wasserabspaltung aus einem entsprechenden Amid erhalten. (J. Amer. Chem. Soc. (1966) 88, 2025-2035).

Gegenstand der Erfindung ist auch ein Verfahren zur Herstellung einer Verbindung der Formel I mit den angegebenen Definitionen, dadurch gekennzeichnet, daß eine Verbindung der Formel I, worin R$^1$ (CONH$_2$) ist, unter Zuhilfenahme eines wasserabspaltenden Mittels in die entsprechende Nitrilform überführt wird, oder daß eine Verbindung der Formel I, worin R eine Bindung und R$^1$ (CONH$_2$) sind mit einem wasserabspaltenden Mittel in eine Verbindung der Formel I, worin R$^1$ die Cyanidfunktion ist, überführt wird und gegebenenfalls nach Entfernen der Schutzgruppen mit einer Verbindung der Formel A-R-X, worin X eine aktivierende Gruppe ist, umgesetzt wird, oder daß eine Verbindung der Formel I, worin R Pro und R$^1$ (CONH$_2$) sind, mit einem wasserabspaltenden Mittel in eine Verbindung der Formel I, worin R$^1$ die Cyanidfunktion ist, überführt wird und nach Abspaltung der Schutzgruppe durch eine Acylierungsreaktion mit einem geschützten, aktivierten Derivat von D-Phe eine Verbindung der Formel I hergestellt wird.

Gegenstand der Erfindung ist ebenfalls ein Verfahren zur Herstellung einer Verbindung der Formel I mit den angegebenen Definitionen, dadurch gekennzeichnet, daß man eine Verbindung der Formel I, worin R$^1$ (CONH$_2$) ist, einer Wasserabspaltungsreaktion an der Carboxamidfunktion unter Zuhilfenahme eines wasserabspaltenden Mittels unterzieht, oder eine Verbindung der Formel I, worin R eine Bindung und R$^1$ (CONH$_2$)

und A eine in der Peptidchemie übliche Schutzgruppe sind, durch eine Wasserabspaltung an der Carboxamidfunktion in die entsprechende Nitriloverbindung überführt und nach Abspaltung der Schutzgruppe mit einer Verbindung der Formel A-R-X, worin X eine aktivierende Gruppe darstellt, zu einer Verbindung der Formel I umsetzt.

Die Carboxyamidfunktion kann auf jeder beliebigen Stufe der Synthese in die Nitrilogruppe überführt werden, d.h. auf der Tripeptid-, Dipeptid- oder Aminosäure-Carboxamidstufe.

Als Ausgangssubstanz wird bevorzugt L-Argininamid, dessen Guanidinogruppe vorzugsweise protoniert ist, eingesetzt. Der peptidaufbau oder die Einführung von Schutzgruppen erfolgt nach Standardverfahren der Peptidchemie, wie sie beispielsweise von M. Bodanszky, Principles of Peptide Synthesis, Springer Verlag, Berlin-Heidelberg-New York- Tokyo 1984, beschrieben werden. Bevorzugt werden die Kupplungsreaktionen in Lösung ausgeführt, indem eine C-terminale Carboxygruppe mit einem Carbodiimid, bevorzugt dem Dicyclohexylcarbodiimid, in Gegenwart einer aziden Verbindung wie 1-Hydroxybenzotriazol aktiviert und mit einer Aminogruppe in Gegenwart einer organischen Base unter Bildung einer Peptidbindung reagieren gelassen wird.

Auf diese Weise werden bevorzugt Boc-D-Phe-Pro, Fmoc-D-Phe-Pro, Z-D-Phe-Pro oder Fmoc-Pro an die Aminogruppe des L-Argininamids gekuppelt.

Neben auf diese Weise hergestellten Argininamidderivaten, die zur Umsetzung in die entsprechenden Nitrile geeignet sind, können auch Argininamidderivate, die mit einer temporären Schutzgruppe an der alpha-Aminogruppe versehen sind, zum Nitril umgesetzt werden. Bevorzugt wird als temporäre Schutzgruppe die Boc-Gruppe benutzt, wobei nach der Wasserabspaltungsreaktion durch Entfernung dieser Schutzgruppe ein kupplungsfähiges Nitril vorliegt, das zu den gewünschten Verbindungen umgesetzt werden kann.

Die Amide werden mit wasserentziehenden Reagenzien in die Nitrile übergeführt beispielsweise mit Thionylchlorid in Dimethylformamid, bevorzugt jedoch mit Phosphoroxchlorid in Pyridin.

In einer besonders bevorzugten Ausgestaltung der Dehydratisierung wird ein mol Amid in Gegenwart von 2 mol Imidazol in pyridin mit 1,1 mol phosphoroxchlorid umgesetzt. Die Reaktionstemperatur wird während der Zugabe von phosphoroxchlorid vorzugsweise im Bereich von -25 bis -15°C gehalten. Die Umsetzung wird dann durch Rühren des Ansatzes während 30 Minuten bis 5 Stunden, bevorzugt einer Stunde, bei Raumtemperatur vervollständigt. Die Produkte werden mittels in der Peptidchemie üblichen Reinigungsmethoden isoliert, wozu vorzugsweise eine Verteilung der Rohprodukte zwischen organischen Lösemitteln und Wasser sowie die Säulenchromatographie dienen.

Die auf diese Weise hergestellten Nitrilderivate zeigten bei den durchgeführten Analysen die korrekte Zusammensetzung. So ließen sich die Nitril-Dreifach-Bindungen im Infrarotspektrum bei etwa 2250 cm$^{-1}$ im erwarteten Bereich auffinden. Kernresonanzuntersuchungen zeigten im $^{13}$C-NMR-Spektrum bei etwa 119 ppm das Vorliegen einer Nitril-Gruppe an.

Im Falle von Nitrilderivaten der Formel I, worin R D-Phe-Pro und A eine Schutzgruppe ist, lassen sich Schutzgruppen vom Urethantyp, bevorzugt sind Boc und Fmoc, mit den üblichen Reagenzien unter Freilegung der N-terminalen Aminogruppe abspalten. Die Boc-Gruppe wird vorzugsweise mit 1,2 N HCl in Eisessig oder mit 50 % Trifluoressigsäure in Methylenchlorid und die Fmoc-Gruppe mit piperidin in Methylenchlorid abgespalten.

Die erfindungsgemäßen Verbindungen zeigen Überraschenderweise eine starke Inhibitorwirkung gegen Serinproteasen. Bevorzugt zeigt sich diese Wirkung gegen Thrombin. Die Spezifität gegenüber weiteren Enzymen, beispielsweise F Xa, wird durch Gruppen am N-Terminus beeinflußt.

Die erfindungsgemäßen Verbindungen eignen sich als Ersatzstoffe natürlich vorkommender Serinproteaseinhibitoren, bevorzugt des AT III. Diese Substanzen und deren physiologisch verträglichen Salze können als Mittel zur Behebung von AT III-Mangel dienen, wodurch ein Thromboserisiko vermindert oder eliminiert wird. Diese Mittel können zusätzlich physiologisch unbedenkliche Träger oder Hilfsstoffe enthalten.

Abkürzungen

AT III    Antithrombin III
Z         Benzyloxycarbonyl
Boc       tert.-Butyloxycarbonyl
Fmoc      9-Fluorenylmethyloxycarbonyl
NMR       Kernmagnetische Resonanz
DC        Dünnschichtchromatographie
$R_F$     Retentionsfaktor
C/T       Chlor/4,4-Bis-(dimethylamino)diphenylmethan-Test
UV        Ultraviolettvisualisation
DCU       Dicyclohexylharnstoff

DCC     Dicyclohexylcarbodiimid
HOBt    Hydroxybenzotriazol
DMF     Dimethylformamid
NMM     N-Methylmorpholin

Laufmittel für Dünnschichtchromatographie:

(A)     n-Butanol/Essigsäure/Wasser 3:1:1
(B)     Chloroform/Methanol/Essigsäure 50:20:5

## Beispiel 1

N(alpha)-Boc-D-phenylalanyl-L-prolyl-L-(1-amino-4-guanidino) valeronitril

1. N(alpha)-Boc-D-phenylalanyl-L-prolyl-L-argininamid

6,6 g Boc-D-Phe-Pro (18 mmol) und 2,43 g HOBt wurden in 100 ml DMF gelöst, auf 0°C gekühlt und mit 3,8 g DCC versetzt. Es wurde 30 Minuten bei 0°C und 30 Minuten bei Raumtemperatur gerührt. Daraufhin wurden 5,28 g Arg-$NH_2$ x 2 $CH_3COOH$ und 3,8 ml NMM zugesetzt. Nach 12 Stunden wurde der ausgefallene DCU abgesaugt, das Lösemittel im Vakuum abgedampft und der Rückstand in Chloroform aufgenommen. Die organische Phase wurde dreimal mit gesättigter Natriumhydrogencarbonatlösung und dreimal mit gesättigter Kochsalzlösung ausgeschüttelt. Die Chloroformlösung wurde über Natriumsulfat getrocknet und im Vakuum eingeengt. Das Peptidderivat wurde durch Eintropfen der konzentrierten Chloroformlösung in Diethylether kristallin gewonnen. Die Kristalle wurden im Hochvakuum über Phosphorpentoxid getrocknet.

Ausbeute:           7,3 g (70 % d. Th.)
Reinheitskontrolle:     DC $R_F$ = 0,46 (A)

2. N(alpha)-Boc-D-Phenylalanyl-L-prolyl-L-(1-amino-4-guanidino) valeronitril

4 g des in 1. hergestellten Amids und 1 g Imidazol wurden in 60 ml Pyridin gelöst. Bei -20°C wurden 2,8 ml Phosphoroxchlorid zugetropft. Der Ansatz wurde dann 1 Stunde bei Raumtemperatur gerührt, im Vakuum eingeengt und der Rückstand in Chloroform aufgenommen.

Die organische phase wurde dreimal mit Wasser extrahiert und über Natriumsulfat getrocknet. Das Peptidderivat wurde durch Eintropfen in Diethylether kristallisiert, mit Ether gewaschen und im Hochvakuum getrocknet.

Ausbeute:           2,6 g (69 % d. Th.)
Reinheitskontrolle:     DC $R_F$ = 0,61 (A)
Schmelzpunkt:       ab 75°C Zersetzung

## Beispiel 2

N(alpha)-Fmoc-D-Phenylalanyl-L-prolyl-L-(1-amino-4-guanidino)valeronitril

1. N(alpha)-Fmoc-D-phenylalanyl-L-prolyl-L-argininamid

1,45 g Fmoc-D-Phe-Pro (3 mmol) und 405 mg HOBt wurden in 40 ml DMF gelöst, bei 0°C mit 630 mg DCC versetzt, anschließend 30 Minuten bei 0°C und 30 min bei Raumtemperatur gerührt. Es wurden dann 880 mg Arg-$NH_2$ x 2 $CH_3COOH$ und 630 µl NMM zugesetzt. Nach 12 Stunden wurde Unlösliches abfiltriert, das DMF abgedampft und der ölige Rückstand in Chloroform aufgenommen. Die organische Phase wurde dreimal mit gesättigter Natriumhydrogencarbonatlösung sowie dreimal mit gesättigter Kochsalzlösung ausgeschüttelt und über Natriumsulfat getrocknet. Nach Abdampfen des Chloroforms wurde ein reines produkt erhalten.

Ausbeute:           900 mg (43 %)
Reinheitskontrolle:     DC $R_F$ = 0,34 (B)

2. N(alpha)-Fmoc-D-phenylalanyl-L-prolyl-L-(1-amino-4 guanidino)valeronitril

770 mg des in 1. synthetisierten produktes wurden in 10 ml pyridin gelöst, mit 100 mg Imidazol versetzt und auf -20°C gekühlt. Es wurden 0,5 ml Phosphorox zugetropft und eine Stunde bei Raumtemperatur gerührt.

Der Ansatz wurde im Vakuum zur Trockene eingedampft und in Chloroform aufgenommen. Diese Lösung wurde mit einer gesättigten Natriumhydrogencarbonatlösung und einer gesättigten Kochsalzlösung je dreimal ausgeschüttelt. Es wurde über Natriumsulfat getrocknet. Nach teilweisem Eindampfen der Lösung im Vakuum wurde das produkt durch Eintropfen in Ether/Ethylacetat (2:1) kristallisiert, abfiltriert und im Hochvakuum getrocknet.

Ausbeute:            380 mg (52,5 %)
Reinheitskontrolle:  DC $R_F$ = 0,62
Schmelzpunkt:        ab 125°C Zersetzung

## Beispiel 3

H-D-phenylalanyl-L-prolyl-L-(1-amino-4-guanidino) valeronitrilditrifluoracetat

270 mg des nach Beispiel 1 hergestellten Produktes wurden mit 0,5 ml Anisol und 3 ml Trifluoressigsäure versetzt und unter Feuchtigkeitsausschluß 5 Minuten bei Raumtemperatur gehalten. Durch Eintropfen in Diethylether wurde das produkt kristallisiert. Es wurde abzentrifugiert, mit Diethylether gewaschen und in Wasser aufgenommen. Die wässrige phase wurde dreimal mit Diethylether extrahiert und gefriergetrocknet. Man erhielt ein weißes lockeres Pulver.

Ausbeute:            170 mg (54 %)
Reinheitskontrolle:  DC $R_F$ = 0,275 (A)

## Funktionsprüfung

Die Wirksamkeit der nach den Beispielen 1, 2 oder 3 hergestellten Substanzen wurde durch Bestimmen der Thrombinzeit ermittelt. Dabei wurde die Konzentration (in µmol/l) ermittelt, die die Thrombinzeit verdoppelt.

## Testausführung

50 µl Inhibitorlösung mit jeweils unterschiedlicher Konzentration,
50 µl Standard-Human-Plasma und
100 µl Diethylbarbitursäure-Natriumacetatpuffer pH 7,6 wurden 45 Sekunden bei 37°C inkubiert und mit
100 µl alpha-Human-Thrombin (3,0 IU/ml) versetzt.
Getestet wurde am Gerät zur Bestimmung der Thrombinzeit von Schnitger & Gross.

## Tabelle 1

| Verbindung nach Beispiel | Verdoppelung der Thrombinzeit bei Konzentration (in µmol/l Endkonz.) |
|---|---|
| 1 | 3,66 |
| 2 | 3,66 |
| 3 | 1,1 |

## Toxizitätsprüfung

Die Toxizität von H-D-Phe-Pro-Arg-CN x 2HCOOH wurde im Tiermodell untersucht. Die Testsubstanz wurde Mäusen i.v. appliziert. Klinisch erkennbare pathologische Befunde wurden ab einer Dosis von 50 mg/kg festgestellt. Die Dosis für eine therapeutische Wirkung sollte bei etwa 1-4 mg/kg liegen.

## Inhibitionskonstante

($K_I$-Werte) von H-D-Phe-Pro-Arg-CN x 2HCOOH:
$K_I$ = 6 x 10$^{-7}$ mol/l (alpha-Human-Thrombin)

**Patentansprüche**

1. Verbindung der Formel I

$$A\text{-}R\text{-}NH\text{-}CHR^1\text{-}(CH_2)_a\text{-}NHC(=NH)NH_2 \quad I$$

worin

$R^1$ die Cyanidfunktion,

R Pro oder D-Phe-Pro,

A ein Wasserstoffatom oder eine in der Peptidchemie übliche Schutzgruppe und

a eine ganze Zahl von 2 bis 5, vorzugsweise 3 oder 4 ist,

sowie ihre physiologisch verträglichen Salze.

2. Verbindung nach Anspruch 1, worin A-R Boc-D-Phe-Pro, Fmoc-D-Phe-Pro, Z-D-Phe-Pro oder Fmoc-Pro ist.

3. D-Phenylalanyl-L-prolyl-L-(1-amino-4-guanidin) valeronitril, seine am N-Terminus mit in der Peptidchemie üblichen Schutzgruppen versehenen Derivaten und seine physiologisch verträglichen Salze.

4. Pharmazeutisches Mittel, dadurch gekennzeichnet, daß es eine Verbindung nach Anspruch 1 sowie einen Träger und gegebenenfalls Hilfs- oder Zusatzstoffe enthält.

5. Verfahren zur Herstellung einer Verbindung der Formel I

$$A\text{-}R\text{-}NH\text{-}CHR^1\text{-}(CH_2)_a\text{-}NHC(=NH)NH_2 \quad I$$

worin

$R^1$ die Cyanidfunktion,

R Pro oder D-Phe-Pro,

A ein Wasserstoffatom oder eine in der Peptidchemie übliche Schutzgruppe und

a eine ganze Zahl von 2 bis 5, vorzugsweise 3 oder 4 ist,

oder eines ihrer physiologisch verträglichen Salze, dadurch gekennzeichnet, daß eine Verbindung der Formel I, worin $R^1$ ($CONH_2$) ist, unter Zuhilfenahme eines wasserabspaltenden Mittels in die entsprechende Nitrilform überführt wird, oder daß eine Verbindung der Formel I, worin R eine Bindung und $R^1$ ($CONH_2$) sind mit einem wasserabspaltenden Mittel in eine Verbindung der Formel I, worin $R^1$ die Cyanidfunktion ist, überführt wird und gegebenenfalls nach Entfernen der Schutzgruppen mit einer Verbindung der Formel A-R-X, worin X eine aktivierende Gruppe ist, umgesetzt wird, oder daß eine Verbindung der Formel I, worin R Pro und $R^1$ ($CONH_2$) sind, mit einem wasserabspaltenden Mittel in eine Verbindung der Formel 1, worin $R^1$ die Cyanidfunktion ist, überfürt wird und nach Abspaltung der Schutzgruppe durch eine Acylierungsreaktion mit einem geschützten, aktivierten Derivat von D-Phe eine Verbindung der Formel I hergestellt wird.

6. Verfahren zur Herstellung einer Verbindung der Formel I, dadurch gekennzeichnet, daß man eine Verbindungt der Formel I, worin $R^1$ ($CONH_2$) ist, einer Wasserabspaltungsreaktion an der Carboxamidfunktion unter Zuhilfenahme eines wasserabspaltenden Mittels unterzieht oder eine Verbindung der Formel I, worin R eine Bindung und $R^1$ ($CONH_2$) und A eine in der Peptidchemie übliche Schutzgruppe sind, durch eine Wasserabspaltung an der Carboxamidfunktion in die entsprechenden Nitriloverbindungen überführt und nach Abspaltung der Schutzgruppe mit einer Verbindung der Formel A-R-X, worin X eine aktivierende Gruppe darstellt, zu einer Verbindung der Formel I umsetzt.

7. Verbindung nach Anspruch 1 zur Verwendung als Antikoagulans.

**Claims**

1. A compound of the formula I

$$A\text{-}R\text{-}NH\text{-}CHR^1\text{-}(CH_2)_a\text{-}NHC(=NH)NH_2 \quad I$$

in which

$R^1$ is the cyanide group,

R is Pro or D-Phe-Pro,

A is a hydrogen atom or a protective group customary in peptide chemistry, and

a is an integer from 2 to 5, preferably 3 or 4, and its physiologically tolerated salts.

2. A compound as claimed in claim 1, in which A-R is Boc-D-Phe-Pro, Fmoc-D-Phe-Pro, Z-D-Phe-Pro or Fmoc-Pro.

3. D-Phenylalanyl-L-prolyl-L-(1-amino-4-guanidino)valeronitrile, its derivatives provided at the N-terminal end with protective groups customary in peptide chemistry, and its physiologically tolerated salts.

4. A pharmaceutical agent which contains a compound as claimed in claim 1 together with a vehicle and, where appropriate, auxiliaries or additives.

5. A process for the preparation of a compound of the formula I

$$A\text{-}R\text{-}NH\text{-}CHR^1\text{-}(CH_2)_a\text{-}NHC(=NH)NH_2 \quad I$$

in which

  $R^1$ is the cyanide group,

  R is Pro or D-Phe-Pro,

  A is a hydrogen atom or a protective group customary in peptide chemistry, and

  a is an integer from 2 to 5, preferably 3 or 4,

or of one of its physiologically tolerated salts, which comprises conversion of a compound of the formula I in which $R^1$ is ($CONH_2$), with the aid of a water-eliminating agent, into the corresponding nitrile form, or comprises conversion of a compound of the formula I in which R is a bond and $R^1$ is ($CONH_2$), using a water-eliminating agent, into a compound of the formula I in which $R^1$ is the cyanide group, and, where appropriate after removal of the protective groups, reaction with a compound of the formula A-R-X in which X is an activating group, or comprises conversion of a compound of the formula I in which R is Pro and $R^1$ is ($CONH_2$), using a water-eliminating agent, into a compound of the formula I in which $R^1$ is the cyanide group, and, after elimination of the protective group, preparation of a compound of the formula I by an acylation reaction with a protected activated derivative of D-Phe.

  6. A process for the preparation of a compound of the formula I, which comprises subjecting a compound of the formula I in which $R^1$ is ($CONH_2$) to a water-eliminating reaction at the carboxamide group with the aid of a water-eliminating agent, or converting a compound of the formula I in which R is a bond and $R^1$ is ($CONH_2$) and A is a protective group customary in peptide chemistry, by water elimination at the carboxamide group, into the corresponding nitrilo compounds and, after elimination of the protective group, reacting with a compound of the formula A-R-X, in which X represents an activating group, to give a compound of the formula I.

  7. A compound as claimed in claim 1 for use as anticoagulant.

## Revendications

  1. Composé de formule I

$$A\text{-}R\text{-}NH\text{-}CHR^1\text{-}(CH_2)_a\text{-}NHC(=NH)NH_2 \quad I$$

dans laquelle

  $R^1$ est la fonction cyanure,

  R est Pro ou D-Phe-Pro,

  A est un atome d'hydrogène ou un groupe protecteur usuel dans la chimie des peptides, et

  a est un nombre entier allant de 2 à 5, de préférence 3 ou 4,

ainsi que ses sels physiologiquement acceptables.

  2. Composé selon la revendication 1, dans lequel A-R est Boc-D-Phe-Pro, Fmoc-D-Phe-Pro, Z-D-Phe-Pro ou Fmoc-Pro.

  3. D-phénylalanyl-L-prolyl-L-(1-amino-4-guanidino)-valéronitrile, ses dérivés munis à l'extrémité N-terminale de groupes protecteurs usuels dans la chimie des peptides, et ses sels physiologiquement acceptables.

  4. Produit pharmaceutique, caractérisé en ce qu'il contient un composé selon la revendication 1 ainsi qu'un véhicule et éventuellement des adjuvants ou additifs.

  5. Procédé pour la préparation d'un composé de formule I

$$A\text{-}R\text{-}NH\text{-}CHR^1\text{-}(CH_2)_a\text{-}NHC(=NH)NH_2 \quad I$$

dans laquelle

  $R^1$ est la fonction cyanure,

  R est Pro ou D-Phe-Pro,

  A est un atome d'hydrogène ou un groupe protecteur usuel dans la chimie des peptides, et

  a est un nombre entier allant de 2 à 5, de préférence 3 ou 4,

ou d'un de ses sels physiologiquement acceptables, caractérisé en ce que l'on convertit en la forme nitrile correspondante un composé de formule I dans lequel $R^1$ est ($CONH_2$), à l'aide d'un agent déshydratant, ou en ce que l'on convertit un composé de formule I dans lequel R est une liaison et $R^1$ est ($CONH_2$), à l'aide d'un agent déshydratant, en un composé de formule I dans lequel $R^1$ est la fonction cyanure, et éventuellement, après élimination des groupes protecteurs, on le fait réagir avec un composé de formule A-R-X, X étant un groupe activateur, ou en ce que l'on convertit un composé de formule I dans lequel R est Pro et $R^1$ est ($CONH_2$), à l'aide d'un agent déshydratant, en un composé de formule I dans lequel $R^1$ est la fonction cyanure, et après élimination du groupe protecteur, on obtient un composé de formule I par une réaction d'acylation avec un dérivé activé protégé de D-Phe.

  6. Procédé pour la préparation d'un composé de formule I, caractérisé en ce que l'on soumet un composé de formule I, dans lequel $R^1$ est ($CONH_2$), à une réaction de déshydratation au niveau de la fonction carboxamide, à l'aide d'un agent déshydratant, ou on convertit en les composés nitrilo correspondants un composé de formule I dans lequel R est une liaison et $R^1$ est $(CONH_2)$ et A est un groupe protecteur usuel dans la chimie des peptides, par une élimination d'eau au niveau de la fonction carboxamide, et après élimination du groupe protecteur, on le fait réagir avec un composé de formule A-R-X dans lequel X représente un groupe activateur, pour aboutir à un composé de formule I.

7. Composé selon la revendication 1, pour utilisation en tant qu'anticoagulant.